# EUROPEAN PATENT APPLICATION

(11) **EP 2 436 265 A2**
(43) Date of publication of application: **04.04.2012**
(21) Application number: 11182303.5
(22) Date of filing: 22.09.2011
(51) Int. Cl.: A01N 37/16, A01N 59/00, A01P 1/00, A61L 2/18, D06L 1/12

(54) **Disinfective cleaning composition**

(30) Priority: 29.09.2010 CH 15872010
(71) Applicant: Coop Genossenschaft, 4053 Basel (CH)
(72) Inventor: Chen, Shan-Jia, 8544 Attikon (CH); Wirz, Irene, 8492 Wila (CH); Müller, Beat, 8153 Rümlang (CH); Lattmann, Ursula, 8618 Oetwil am See (CH)
(74) Representative: Braun, André jr.

(57) **Abstract**

The present invention is directed to a disinfective cleaning composition comprising disinfective and cleaning components which together preserve sensitive materials, in particular textiles over many treatment cycles. Furthermore, the invention relates to a method for cleaning and/or disinfecting objects, preferably textiles by bringing them into contact with the inventive compositions as well as corresponding uses of the compositions and methods.

## Description

The present invention is directed to a disinfective cleaning composition comprising disinfective and cleaning components which together preserve sensitive materials, in particular textiles over many treatment cycles. Furthermore, the invention relates to a method for cleaning and/or disinfecting objects, preferably textiles by bringing them into contact with the inventive compositions as well as corresponding uses of the compositions and methods.

### Background of the invention

Disinfective compositions as well as disinfective cleaning compositions that contain peracetic acid, hydrogen peroxide, acetic acid and optionally further disinfective or cleaning agents are state of the art.

WO 2004/108171 A1 relates to aqueous disinfecting compositions based on peracetic acid, hydrogen peroxide, peroxyacids, acetic acid and containing less than 0.15 vol.-% peracetic acid as well as a stabilizer formed from a mixture of non-oxidized essential oils and triacids for use in neurology, ophthalmology and contact lenses.

US 6,168,808 B1 describes disinfective compositions containing peracetic acid, acetic acid, hydrogen peroxide, an amine oxide and optionally a non-ionic surfactant.

WO 2005/001011 A1 teaches antimicrobial and antiviral compositions comprising a combination of specific non-ionic surfactants together with amphoteric and/or anionic surfactants.

US 6,257,253 B1 is directed to sanitizing and destaining compositions for dishware, kitchenware and tableware products containing peroxycarboxylic acids such as peracetic acid, a carboxylic acid such as acetic acid and hydrogen peroxide.

US 2010/0076082 A1 discloses a disinfective composition for use in dialysis machines that comprises peracetic acid, hydrogen peroxide and acetic acid as well as concentrated nitric acid, non-ionic surfactant, amine oxide and stabilizer. While useful for stainless steel and inert tubing, the composition is too harsh for use on more sensitive materials such as textiles because of the concentrated nitric acid and the amine oxide.

Textiles are often produced in developing countries, are transported or stored under hygiene conditions that may lead to microbial contamination. Next to contamination humid and warm environmental conditions assist propagation of the microbial load to a concentration critical for human health. Moreover, many textiles require frequent washes and disinfections, for example, clothing for medical personnel, towels and linen in hotels, hospitals, etc. However, textiles and other sensitive materials are prone to deteriorate in quality, integrity and colour with every wash and/or disinfection step.

It is the objective of the present invention to provide an improved disinfective cleaning composition, in particular one that effectively kills microorganisms, one that is preferably environmentally harmless and biologically degradable, and one which does not harm sensitive materials such as textiles even after repeated treatments.

In a first aspect the above objective is solved by a disinfective cleaning composition comprising:
0.0001 to 5, preferably 0.0005 to 3 wt.-% peracetic acid,
0.0005 to 5, preferably 0.002 to 3 wt.-% hydrogen peroxide,
0.001 to 5, preferably 0.002 to 3 wt.-% acetic acid,
0.001 to 5, preferably 0.01 to 3 wt.-% non-ionic surfactant(s),
0.001 to 5, preferably 0.01 to 3 wt.-% detergent(s),
0.001 to 3, preferably 0.01 to 2 wt.-% anionic surfactant(s) and
0.0001 to 3, preferably 0.001 to 2 wt.-% chelate compound NR₁R₂R₃
   (R₁=R₂=CH₂COOY, R₃=CHXCOOY, X=H or CH₃, Y=H or alkali, preferably H, Na or K)
   and water.

The above aqueous composition is for direct use and contact with objects in need of being cleaned and disinfected. The water content of the composition of the invention will vary with the content of the other components in the composition. All components will add up to 100 wt.-%.

Alternatively, the above aqueous composition can be prepared, transported and/or stored until use as a liquid or solid concentrate.

In a preferred embodiment the invention relates to a liquid or solid concentrate of the disinfective cleaning composition of the invention, preferably comprising:
0.1 to 10, preferably 0.5 to 5 wt.-% peracetic acid,
0.2 to 60, preferably 0.5 to 40 wt.-% hydrogen peroxide,
0.2 to 10, preferably 0.5 to 8 wt.-% acetic acid,
1 to 60, preferably 2 to 25 wt.-% non-ionic surfactant(s),
1 to 30, preferably 2 to 20 wt.-% detergent(s),
1 to 40, preferably 2 to 20 wt.-% anionic surfactant(s),
0.1 to 15, preferably 1 to 10 wt.-% chelate compound NR₁R₂R₃ (R₁=R₂=CH₂COOY, R₃=CHXCOOY, X=H or CH₃, Y=H or alkali, preferably H, Na or K), and preferably water.

The chelate compound NR₁R₂R₃ for preparing the composition or concentrate of the invention is preferably the tetra sodium salt of N,N-diacetic acid glutamic acid. It is, for example, available as Dissolvine^{®} GL-47-S from Akzo Nobel.

The concentrate is preferably an aqueous liquid concentrate.

The terms "peracetic acid", "hydrogen peroxide" and "acetic acid" are meant to include all physiologically acceptable salts and precursors thereof.

Non-ionic surfactant(s) for use in the inventive composition or concentrate are preferably selected from the group consisting of oxo fatty alcohols featuring a 7 C to 20 C chain with 3 to 10 ethoxylates, preferably oxo fatty acid (FA) (C9-13) EO/PO, oxo fatty acid (C9/11) 6EO, oxo fatty acid (C12/18) EO/BO, decyl polyglucoside).

Anionic surfactant(s) for use in the inventive composition or concentrate are preferably selected from the group consisting of alkyl benzene sulfonic acid, alkali alkyl benzene sulfonate, alkyl sulfonic acid, alkali alkyl sulfonate, alkali alkyl sulfate, alkali alkenyl sulfate, fatty acid ether sulfate and fatty alcohol sulfonate, preferably alkyl benzene sulfonic acid, alkali alkyl benzene sulfonate, alkyl ether sulfonic acid and alkali alkyl ether sulphate, more preferably cumene sulfonate and laureth sulfate with 2 EO.

The term "detergent" as used herein relates to alkali salts, preferably sodium and/or potassium salts of fatty acids. A preferred detergent for use in the present invention is the alkali salt of palm coconut acid, preferably its sodium salt.

It was surprisingly found that the combination of the disinfective components peracetic acid, hydrogen peroxide and acetic acid together with the cleaning components non-ionic surfactant(s), anionic surfactant(s), detergent(s) and chelator does not only have the additive effects of efficient disinfection and cleaning but actually preserves the physical properties of sensitive materials, in particular textiles of natural and/or synthetic origin, much better than the cleaning components when used by themselves. In short, the combination reduces damage to sensible materials that results form multiple washes/disinfections. This is demonstrated experimentally below in example 6 (textile preservation). The inventive two component disinfective (A) and cleaning (B) composition (A+B) was tested in comparison to the cleaning component B alone for its impact on textiles, in particular on fiber damage, with standardized EMPA test cloth 300 at 40 °C in two identical washing machines, one used with both components A and B and the other used with the cleaning component B only. Textile wear was tested after runs 1, 25 and 50. The degree of polymerization (viscosity) was tested after run 1 and 25. The combination of the cleaning and disinfective components of the present invention demonstrate a protective effect on sensible textiles with respect to the maintenance of tensile strength and viscosity over many wash cycles.

In a preferred embodiment the present invention relates to a concentrate of the disinfective cleaning composition of the invention comprising at least two components:
component A comprising:
   0.1 to 10, preferably 0.5 to 5 wt.-% peracetic acid,
   0.2 to 60, preferably 0.5 to 40 wt.-% hydrogen peroxide,
   0.2 to 10, preferably 0.5 to 8 wt.-% acetic acid and
      as well as
component B comprising:
   1 to 60, preferably 2 to 25 wt.-% non-ionic surfactant(s),
   1 to 30, preferably 2 to 20 wt.-% detergent(s),
   1 to 40, preferably 2 to 20 wt.-% anionic surfactant(s),
   0.1 to 15, preferably 1 to 10 wt.-% chelate compound NR₁R₂R₃ (R₁=R₂=CH₂COOY, R₃=CHXCOOY, X=H or CH₃, Y=H or alkali, preferably H, Na or K),
   wherein one or both components preferably comprise water and are combined before or during use.

Preferably at least component A comprises water, more preferably both components A and B comprise water.

The two-component concentrate embodiment allows for separate storage and also for separate use of the components. The components can be used separately and be combined if a preserving cleaning and disinfecting activity is desired. For example, if a disinfecting action is required less often than a cleaning action the disinfection component can be saved for later use. The two-component system is more economical and can be more effective. On the other hand, using both components is better for preserving the properties of sensitive materials. The two-component system can allow a stable system of the detergent component, reactive components and the disinfective components, the combination of the two components during the application process can deliver a high performance of the demand function.

Of course, all substances contained in the inventive concentrates or their components A and B add up to 100 wt.-%. It is noted that the above concentrate compositions and components A and B thereof need to be further diluted with aqueous solvents, preferably water only - depending on the treatment mode, treatment conditions and treatment materials.

The disinfective cleaning composition for direct use of the invention does not require nitric acid and/or amine oxides for an effective disinfection. Furthermore, both compounds are rather aggressive and should be omitted. In a preferred embodiment the disinfective cleaning composition and concentrate of the invention does not comprise nitric acid and/or amine oxides.

The compositions, concentrates and components thereof of the present invention preferably additionally comprise at least one additive selected from the group consisting of thickener, solutizer, bleaching agent, bleach activator, bleach stabilizer, enzymes, builders / water softeners, optical brighteners, anti-foaming agents, greying inhibitors, corrosion inhibitors, antimicrobial and antiviral compounds (next to those in component A).

However, for reasons of economy, health, environmental protection and the efficacy of the synergistic cleaning and disinfecting activity of the essential components it is more preferred that the composition, concentrate and components thereof of the present invention are essentially devoid, preferably totally devoid of one or more, preferably all of the substances selected from the group consisting of alkylated phenols and alkylated phenolic derivatives, halogenated compounds, preferably chlorinated compounds, aromatic carbon hydrates, quaternary ammonium compounds, cationic surfactants, phosphate and phosphonate compounds, optical brighteners, enzymes, organic silicon compounds, dyes, allergic perfumes according to the criteria of 76/768/EWG, preservatives, organic polymers, preferably selected from polyvinyl pyrrolidon, polycarboxylate and CMC (carboxymethyl cellulose), heavy metals, preferably copper, cobalt and nickel, and bleach activators, preferably TAED (tetraacetyl ethylene diamine), TAGU (N,N', N" ,N"'-tetraacetyl glycol uryl).

In a preferred embodiment the total VOC (volatile organic compound) content in the composition, the concentrate and its components A and B is less than 3 wt.-%. Typical volatile compounds in cleaning and/or disinfecting compositions are, for example ethanol, isopropyl alcohol, n-propyl alcohol, acetic acid, etc.

In a preferred embodiment the composition, concentrates and components of the present invention can be biodegraded, preferably biodegraded according to the test method OECD 302B, and more preferably reach a DOC decomposition of over 96 % after 9 days and over 98 % after 21 days. It is also preferred that for the compositions, concentrates and components of the present invention the nitrification data is more than 75 mg/l after 4 days and/or the mineralization is more than 75 % after 9 days and more preferably more than 90 % after 28 days.

In a most preferred embodiment, the concentrate composition of the present invention comprises, preferably as component A:
about 3.0 wt.-% peracetic acid, about 30.0 wt.-% hydrogen peroxide,
about 3.0 wt.-% acetic acid and
   preferably as component B:
about 20 wt.-% non-ionic surfactant(s), about 7 wt.-% anionic surfactant(s),
about 12 wt.-% detergent, preferably palm coconut acid or more preferably its sodium salt, about 2.5 wt.-% chelate compound NR₁R₂R₃ (R₁=R₂=CH₂COOY, R₃=CHXCOOY, X=H or CH₃, Y=H or
alkali, preferably H, Na or K) and
optionally as well as preferably contained in component B:
   about 2.5 wt.-% triethanol amine,
   about 9.0 wt.-% solvent PEG-8,
   about 2.0 wt.-% trisodium citrate,
   about 1.0 wt.-% glycerin and/or
   about 3.0 wt.-% ethanol and
   water.

The water may be present in component A and/or B, preferably in both components.

It is noted that any of the above optional and preferred compounds, the triethanol amine, PEG-8, trisodium citrate, glycerin and ethanol could also be contained in component A or even in a further separate component.

The term "about" as used above is meant to indicate a number variation of less than or equal 20 %, preferably less than or equal 10 %, more preferably less than or equal 5 %, most preferable a variation in the last digit of + 1.

For cleaning and disinfection the composition of the present invention as such or in the form of a concentrate, e.g. as concentrate components A and B (added separately and combined during use), is brought into contact with the object(s) to be treated. Typically, the concentrate is diluted before and/or during contact with water to form the inventive aqueous composition featuring effective concentrations of at least all essential substances. However, the aqueous composition of the invention may also comprise other substances, e.g. organic compounds such as PEG-8, glycerin, ethanol, triethanol amine, salts such as trisodium citrate, etc. in an amount that assists good product stability, that can improve the cleaning activity or at least does not impair disinfecting activity. In a preferred embodiment the present invention is directed to a composition of the invention comprising an above concentrate of the invention in a concentration of 0.5 to 100 g/L, preferably 1 to 50 g/L, more preferably 2 to 30 g/L, most preferably 2 to 20 g/L aqueous solvent, preferably water.

In a further aspect, the present invention is directed to a method for cleaning and/or disinfecting objects, preferably textiles made of synthetic and/or natural materials, wherein the objects are brought into contact with the disinfective cleaning composition or diluted concentrate of the invention in an effective concentration, at a temperature and for a time suitable for cleaning and/or disinfecting said objects.

The term "object" as used herein is meant to encompass any material object that can be cleaned and disinfected without being affected by substantial damage. For example, water soluble objects or objects that react essentially with one or more substances of the compositions of the invention are not suitable for the method. Preferred objects for practicing the method of the invention comprise or are made of textiles of natural and/or synthetic origin, in woven and non-woven form, e.g. clothes, linen, towels, shoe ware, table cloths, drapes, bags, etc. However, the compositions of the present invention are not limited to textile materials. They can also be used to clean and disinfect non-textile objects and materials, e.g. medical devices and equipment, kitchenware, dishware and tableware, cleaning technology etc.

In a most preferred embodiment the method of the invention is one for cleaning and disinfecting textiles, preferably using a washing machine.

In a preferred embodiment the contact temperature of the (aqueous) disinfective cleaning composition or diluted concentrate is 0 to 80, preferably 15 to 50, more preferably 20 to 45°C.

In a further preferred embodiment the objects to be treated are brought into contact with said concentrate in a concentration of 0.5 to 100 g/L, preferably 1 to 50 g/L, more preferably 2 to 30 g/L, most preferably 2 to 20 g/L aqueous solvent, preferably water.

In another preferred embodiment the time of contact between the object and the disinfective cleaning composition or diluted concentrate is at least 1 second, preferably at least 1, 5 or 20 seconds to at most 2, 3, 5, 10, 30 or 60 minutes, more preferably at least 1 second to 10 minutes or 5 seconds to 5 minutes, most preferably 5 seconds to 3 minutes or 20 seconds to 2 minutes. It is noted that the disinfecting efficacy of the disinfective cleaning composition/diluted concnetrate is enormous, particularly for a virus load, more particular for killing the virus H1N1. For killing said virus efficiently, 1 or a view seconds of contact will suffice.

In a more preferred embodiment component A of the concentrate is used in a concentration of 0.2 to 20, preferably 0.5 to 15, more preferably 5 to 12 g/L and component B of the concentrate is used in a concentration of 1 to 30, preferably 2 to 15 g/L.

In a most preferred embodiment of the inventive method it is used for killing virus, preferably for killing virus H1N1, on objects, preferably textiles made of synthetic and/or natural materials, preferably wherein
(i) the contact temperature of the disinfective cleaning composition or diluted concentrate is 0 to 50, more preferably 5 to 30, most preferably 10 to 25°C, and/or
(ii) the time of contact between the object and the disinfective cleaning composition or diluted concentrate is at least 1 to 10 minutes, preferably 5 seconds to 3 minutes, most preferably 20 seconds to 2 minutes.

In a third aspect the present invention relates to the use of a disinfective cleaning composition and/or concentrate of the invention and/or a method of the invention for cleaning and/or disinfecting objects, preferably textiles made of synthetic and/or natural materials, most preferably for killing virus, preferably the virus H1N1.

In the following the subject-matter of the invention will be described in more detail referring to specific embodiments which are not intended to be construed as limiting to the scope of the invention.

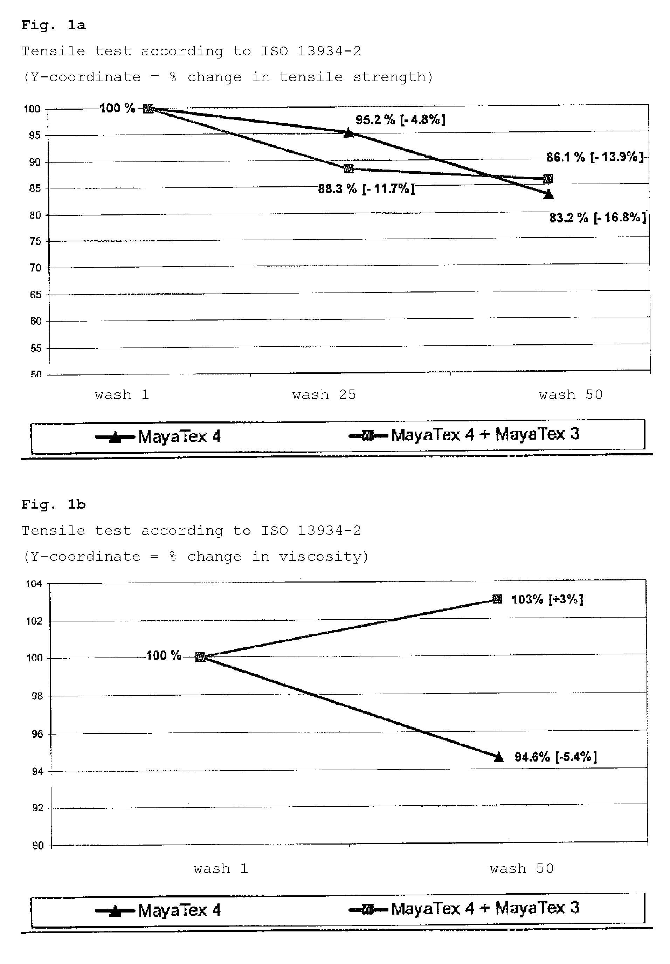

- Fig. 1a: shows the change in tensile strength after washes 1, 25 and 50 with MayaTex4 (cleaning component) alone vs MayaTex4 and MayaTex 3 (cleaning and disinfective components).
- Fig. 1b: depicts the change in viscosity after 50 washes with MayaTex4 (cleaning component) alone vs MayaTex4 and MayaTex 3 (cleaning and disinfective components).

### Examples

### Example 1 - Materials

Several disinfective cleaning compositions according to the present invention were prepared by combining its disinfecting and its cleaning components.

Concentrate 1 is a concentrate for preparing a composition according to the invention. It was used for the experiments reported in the following examples 2 to 6.

Concentrate 1:
component A (MayaTex 3)
   about 3.0 wt.-% peracetic acid,
   about 30.0 wt.-% hydrogen peroxide,
   about 3.0 wt.-% acetic acid and
   water;
component B (MayaTex 4):
   about 20 wt.-% non-ionic surfactant: (5.2 % oxo fatty acid (C9-13) EO/PO,
   5% oxo fatty acid (C9/11) 6EO, 7% oxo fatty acid (C12/18) EO/BO, 2% decyl polyglucoside),
about 7 wt.-% anionic surfactant: (4 % sodium cumene sulfonate and 3 % sodium laureth sulfate with 2 EO),
about 12 wt.-% detergent: sodium salt of palm coconut acid, about 2.5 wt.-% chelate compound NR₁R₂R₃ (R₁=R₂=CH₂COONa, R₃=CHXCOONa, X=H or CH₃), and
   water.

The following are particular embodiments of further concentrates for preparing compositions of the present invention which have also been successfully tested for disinfective and cleaning properties.

Concentrate 2:
component A (MayaTex 3)
   about 5.0 wt.-% peracetic acid,
   about 25.0 wt.-% hydrogen peroxide,
   about 3.0 wt.-% acetic acid and
component B (MayaTex 4) :
   about 22 wt.-% non-ionic surfactant: (7 % oxo fatty acid (C9-13) EO/PO,
   4% oxo fatty acid (C9/11) 6EO, 8% oxo fatty acid (C12/18) EO/BO, 3% decyl polyglucoside),
   about 5 wt.-% anionic surfactant: (3 % sodium cumene sulfonate and 2 % sodium laureth sulfate with 2 EO),
   about 8 wt.-% detergent: sodium salt of palm coconut acid, about 3.9 wt.-% chelate compound NR₁R₂R₃ (R₁=R₂=CH₂COONa, R₃=CHXCOONa, X=H or CH₃), and
   water.

Concentrate 3:
component A (MayaTex 3)
   about 5.5 wt.-% peracetic acid,
   about 26 wt.-% hydrogen peroxide,
   about 2.0 wt.-% acetic acid and
component B (MayaTex 4) :
   about 16 wt.-% non-ionic surfactant: (4 % oxo fatty acid (C9-13) EO/PO, 4 % oxo fatty acid (C9/11) 6EO, 6 % oxo fatty acid (C12/18)
      EO/BO, 2% decyl polyglucoside),
      about 10 wt.-% anionic surfactant: (4 % sodium cumene sulfonate and 6 % sodium laureth sulfate with 2 EO),
      about 8 wt.-% detergent: sodium salt of palm coconut acid, about 5 wt.-% chelate compound NR₁R₂R₃ (R₁=R₂=CH₂COONa, R₃=CHXCOONa, X=H or CH₃), and
      water.

Components A and B were combined and diluted directly before use to result in 20 g/L component A and 4 g/L component B in regular tap water.

### Example 2 - Chemothermic wash disinfection / antiviral activity

The above disinfectant cleaning composition was tested for its antiviral activity on influenza virus A (H1N1) (swine) in accordance with EN 14476:2007-2 at low virus load and 20 °C.

According to EN 14476:2007-2 a disinfectant is effective if the initial virus titre is reduced by at least 4 log₁₀ numbers, i.e. leading to an inactivation of at least 99.99 %. Contact times were 1 and 2 minutes, respectively. Already after 1 minute contact time a reduction of at least 4 log₁₀ numbers was demonstrated. Based on the test report S10ML1005M dated 18.02.2010 this result was confirmed by Dr. Jochen Steinmann of Mikrolab GmbH, Norderoog 2, DE-28259 Bremen.

### Example 3 - Chemothermic wash disinfection / bacteriocidal and antifungal activity

The above disinfectant cleaning composition was tested for its antibacterial and antifungal activity against *S*. *aureus, E. hirae, P. aeruginosa* and *C*. *albicans* in accordance with
- DGHM/VAH-method no. 7 "Bestimmung der bakteriostatischen und fungistatischen Wirkung sowie geeigneter Neutralisierungsmittel",
- DGHM/VAH-method no. 8 "Bestimmung der bakterioziden und fungiziden Wirkung im qualitativen Suspensionsversuch",
- DGHM/VAH-method no. 9.1 with increased load "Bestimmung der bakterioziden und fungiziden Wirkung im quantitativen Suspensionsversuch",
- DGHM/VAH-method no. 17.1 "Chemothermische Wäschedesinfektioon - Einbadverfahren nach DIN 11905 mit Desinfektion vor dem ersten Ablassen der Flotte (praxisnaher Versuch)"
in the version of September 1^{st}, 2001.

The oriented investigation in accordance with DGHM/VAH-method no. 7 demonstrated that when using a combination made of 10 % horse blood and 20,000 units catalase per ml an effective neutralizing agent was found. The results of the qualitative suspension tests demonstrate that *P*. *mirabilis* and *E*. *coli* do not have a higher resistency compared to the test organism *P*. *aeroguinosa* required for the quantitative suspension test.

The results of the quantitative suspension tests under severe conditions with higher organic load employing the above disinfectant cleaning composition in a washing machine at 40 °C and a liquor ratio of 1:5 demonstrate within 20 minutes treatment the following log₁₀ reduction factors:
7.37 for *S*. *aureus;* 6.43 for *E*. *hirae*; 6.92 for *P*. *aeruginosa* and 5.64 for *C*. *albicans* compared to log₁₀ germ reduction rates without the composition between 3.49 and 4.70 units.

Moreover, in further utility tests under more severe conditions with addition of 12.5 ml blood per kilogram laundry log₁₀ reduction factors were:
7.51 for *S*. *aureus;* 7,82 for *E*. *hirae*; 8.03 for *E*. *coli* and 6.85 for *C*. *albicans* compared to log₁₀ germ reduction rates without the composition between 3.49 and 4.70 units.

Based on the test report MB 4587/08 of the wfk-Intitut für Angewandte Forschung GmbH of November 25, 2008 the above results were confirmed by Prof. Dr. J. Krämer, Am dickem Stein 25, DE-53913 Swisstal-Heimerzheim.

### Example 4 - Chemothermic wash disinfection / bacteriocidal and antifungal activity

The above disinfectant cleaning composition was tested in accordance with the requirement "17.2 Chemothermosche Wäschdesinfektion" (dated September 2001) of the Deutsche Gesellschaft für Hygiene und Mikrobiologie resulting in test reports SN 8539 and SN 8540 of March 27, 2009.

The results of the quantitative suspension tests at 40 °C indicate log₁₀ reduction factors of at least 6.32 units within 15 minutes.

Moreover, further utility tests under more severe conditions with addition of 12.5 ml blood per kilogram laundry at a liquor ratio of 1:5 show log₁₀ reduction factors of at least 7.06 for bacteria and at least 6.27 for *C*. *albicans.*

Prof. Dr. H.-P. Werner, HygCen GmbH, Börnhövedstr. 78, DE-19055 Schwerin confirmed these results.

### Example 5 - Chemothermic wash disinfection / biocompatibility

The cleaning component B of the above composition was investigated for biocompatibility by testing biological elimination, mineralization and inhibition of nitrification. The method used was OECD Guideline 302B with investigation of mineralization, standard operation procedure EMPA (SOP); 4 L sample. The origin of the live sludge was ARA Bruggen (25.02.2003), its concentration 0.2 g dry substance/L; activity surveillance of the life sludge was done by means of ethylene glycol, minimal elimination of 95 % after 7 days, surveillance of elimination was done by determination of solvated organic carbon (DOC), surveillance of mineralization was done by titrimetric determination of carbonate in absorption liquor, initial concentration of 156 mg product/L, 48.5 mg TOC/L.

The results for elimination, mineralization and nitrification are provided in the tables below:

**Table 1 Elimination and mineralization data**

| Duration t = | 3 h | 1 d | 4 d | 9 d | 14 d | 21 d | 28 d |
|---|---|---|---|---|---|---|---|
| DOC in sample* | 46.9 | 33.5 | 11.4 | 6.1 | 5.2 | 3.9 | 3.6 |
| DOC in reference* | 5.1 | 5.5 | 5.2 | 4.1 | 3.4 | 3.0 | 3.3 |
| Difference* | 41.8 | 28.0 | 6.2 | 2.0 | 1.8 | 0.9 | 0.3 |
| *Elimination in* % | *14* | *42* | *87* | *96* | *96* | *98* | *99* |
| CO2-C in sample | | | 150.8 | 272.0 | 320.0 | 341.2 | 356.5 |
| CO2-C in reference | | | 75.0 | 124.1 | 149.9 | 169.7 | 183.5 |
| Difference [mg] | | | 75.8 | 147.9 | 170.1 | 171.5 | 173.0 |
| Mineralization % | | | 39 | 77 | 88 | 89 | 90 |

| | | | | | | | |
|---|---|---|---|---|---|---|---|
| * [mg/L] | | | | | | | |

**Table 2**

| Treatment time t | 3 hours | 1 day |
|---|---|---|
| NH₄-N** test sample | 5.0 | 0.2 |
| NH₄-N** reference sample | 5.9 | < 0.1 |

**Method used: life sludge treatment as for determining the elimination on a DOC basis. Determination of the ammonium concentration by means of the Berthelot reaction, method 30 of the Guidelines of the Swiss Department of Internal Affairs "Richtlinien des EDI ("Eigenössisches Department des Innern").

In its test report dated 28.03.2003 the EMPA St. Gallen, Lerchenfeldstrasse 5, CH-9014-St. Gallen confirmed that component B features a bioelimination of 96 % after 14 days, a mineralization of 90 % after 28 days and that it is biologically well degradable. In a concentration of 156 mg/L component B did not impair the sensitive nitrification.

### Example 6 - Chemothermic wash disinfection / textile preservation

The above two component disinfective cleaning composition (A+B) was tested in comparison to the cleaning component B by the Swiss Textile Testing Institute TESTEX, Gotthardstrasse 61, CH-8027 Zurich, for its impact on textiles, in particular on fiber damage. Textile wear was tested after runs 1, 25 and 50. The degree of polymerization (viscosity) was tested after runs 1 and 25.

50 washes were performed with standardized EMPA test cloth 300 at 40 °C in two identical washing machines, one used with both components A and B and the other used with the cleaning component B only.

The results are provided below.

**Table 3**

| | MayaTex 3 | MayaTex 4 | MayaTex 3 | MayaTex 4 | MayaTex 3 | MayaTex 4 |
|---|---|---|---|---|---|---|
| | | MayaTex 3 | | | | |
| WASH TEST*** | | | | | | |
| washes | 1 | 1 | 25 | 25 | 50 | 50 |
| Dosage | 20 g | 20+100 | 20 g | 20+100 | 20 g | 20+100 |
| Liquor ratio | 1 : 5 | g | 1 : 5 | g | 1 : 5 | g |
| Treatment | 20 min | 1 : 5 | 20 min | 1 : 5 | 20 min | 1 : 5 |
| time | 40 °C | 20 min | 40 °C | 20 min | 40 °C | 20 min |
| Water temp. | 2 kg | 40 °C | 2 kg | 40 °C | 2 kg | 40 °C |
| Total load | | 2 kg | | 2 kg | | 2 kg |
| TENSILE | | | | | | |
| TEST** | 5 | 5 | 5 | 5 | 5 | 5 |
| Measurements | 77.8 | 78.3 | 74.1 | 69.1 | 64.7 | 67.4 |
| * | 4.65 | 5.48 | 3.31 | 5.10 | 3.66 | 3.74 |
| tensile | 14.8 | 14.7 | 17.1 | 17.0 | 17.5 | 18.3 |
| strength* | 2.35 | 3.31 | 2.38 | 2.51 | 1.97 | 0.59 |
| CV% t. | | | | | | |
| strength* | | | | | | |
| elongation* | | | | | | |
| CV% | | | | | | |
| elongation* | | | | | | |
| VISCOSITY*** | | | | | | |
| * | 2 | 2 | | | 2 | 2 |
| measurements | 10.11 | 9.78 | | | 9.57 | 10.08 |
| viscosity | 1537 | 1487 | | | 1454 | 1532 |
| polymerization | low | low | | | low | low |
| damage | | | | | | |

| | | | | | | |
|---|---|---|---|---|---|---|
| * chain / longitudional; **(ISO 13934-1 (dry); ***(TESTEX method, drying mode/hanging); **** SN 195 598 (EWN) | | | | | | |

The results are graphically summarized in Figs. 1a and 1b. Fig. 1a shows the change in tensile strength after washes 1, 25 and 50 with MayaTex4 (cleaning component) alone vs MayaTex4 and MayaTex 3 (cleaning and disinfective components). After 25 washes the combination leads to a slightly higher loss of tensile strength of the tested textile than for the cleaning component alone. However, over the subsequent 25 (total of 50) washes the disinfective component protects from further loss of tensile strength and the tensile strength achieved with the cleaning component alone becomes less than with the combination of cleaning and disinfective components.

Fig. 1b depicts the change in viscosity after 50 washes with MayaTex4 (cleaning component) alone vs MayaTex4 and MayaTex3 (cleaning and disinfective components). After 50 washes the combination actually increases viscosity whereas the cleaning component alone leads to a loss of viscosity of the tested textile.

It is evident that the combination of the cleaning and disinfective components of the present invention have a protective effect on textiles with respect to the maintenance of tensile strength and viscosity over many wash cycles.

## Claims

1. Disinfective cleaning composition comprising:
0.0001 to 5, preferably 0.0005 to 3 wt.-% peracetic acid,
0.0005 to 5, preferably 0.002 to 3 wt.-% hydrogen peroxide,
0.001 to 5, preferably 0.002 to 3 wt.-% acetic acid,
0.001 to 5, preferably 0.01 to 3 wt.-% non-ionic surfactant(s),
0.001 to 5, preferably 0.01 to 3 wt.-% detergent(s),
0.001 to 3, preferably 0.01 to 2 wt.-% anionic surfactant(s) and
0.0001 to 3, preferably 0.001 to 2 wt.-% chelate compound NR₁R₂R₃
(R₁=R₂=CH₂COOY, R₃=CHXCOOY, X=H or CH₃, Y=H or alkali, preferably H, Na or K)
and water.

2. Concentrate of a disinfective cleaning composition according to claim 1 comprising:
0.1 to 10, preferably 0.5 to 5 wt.-% peracetic acid,
0.2 to 60, preferably 0.5 to 40 wt.-% hydrogen peroxide,
0.2 to 10, preferably 0.5 to 8 wt.-% acetic acid,
1 to 60, preferably 2 to 25 wt.-% non-ionic surfactant(s),
1 to 30, preferably 2 to 20 wt.-% detergent(s),
1 to 40, preferably 2 to 20 wt.-% anionic surfactant(s),
0.1 to 15, preferably 1 to 10 wt.-% chelate compound NR₁R₂R₃ (R₁=R₂=CH₂COOY, R₃=CHXCOOY, X=H or CH₃, Y=H or alkali, preferably H, Na or K), and preferably water.

3. Concentrate according to claim 2 comprising at least two components:
component A comprising:
0.1 to 10, preferably 0.5 to 5 wt.-% peracetic acid,
0.2 to 60, preferably 0.5 to 40 wt.-% hydrogen peroxide,
0.2 to 10, preferably 0.5 to 8 wt.-% acetic acid as well as component B comprising:
1 to 60, preferably 2 to 25 wt.-% non-ionic surfactant(s),
1 to 30, preferably 2 to 20 wt.-% detergent(s),
1 to 40, preferably 2 to 20 wt.-% anionic surfactant(s) and 0.1 to 15, preferably 1 to 10 wt.-% chelate compound NR₁R₂R₃ (R₁=R₂=CH₂COOY, R₃=CHXCOOY, X=H or CH₃, Y=H or alkali, preferably H, Na or K)
wherein one or both components optionally comprise water and are combined before or during use.

4. Concentrate according to claims 2 or 3 comprising preferably as component A:
about 3.0 wt.-% peracetic acid, about 30.0 wt.-% hydrogen peroxide,
about 3.0 wt.-% acetic acid and
preferably as component B:
about 20 wt.-% non-ionic surfactant(s), about 7 wt.-% anionic surfactant(s),
about 12 wt.- % detergent, preferably palm coconut acid or
more preferably its sodium salt, about 2.5 wt.-% chelate compound NR₁R₂R₃ (R₁=R₂=CH₂COOY, R₃=CHXCOOY, X=H or CH₃, Y=H or
alkali, preferably H, Na or K) and
optionally as well as preferably contained in component B
about 2.5 wt - % triethanol amine,
about 9.0 wt -% solvent PEG-8,
about 2.0 wt - % trisodium citrate,
about 1.0 wt - % glycerin,
about 3.0 wt - % ethanol and
water.

5. Disinfective cleaning composition according to claim 1 or concentrate according to any one of claims 2 to 4 essentially devoid of one or more, preferably all of the substances selected from the group consisting of alkylated phenols and alkylated phenolic derivatives, halogenated compounds, preferably chlorinated compounds, aromatic carbon hydrates, quaternary ammonium compounds, cationic surfactants, phosphate and phosphornate compounds, optical brighteners, enzymes, organic silicon compounds, dyes, allergic perfumes according to the criteria of 76/768/EWG, preservatives, organic polymers, preferably selected from polyvinyl pyrrolidon, polycarboxylate and CMC (carboxymethyl cellulose), heavy metals, preferably copper, cobalt and nickel, and bleach activators, preferably TAED (tetraacetyl ethylene diamine), TAGU (N,N', N'',N'''-tetraacetyl glycol uryl).

6. Disinfective cleaning composition or concentrate according to any of claims 1 to 5, wherein the total VOC (volatile organic compound) content in the composition is less than 3 wt.-%.

7. Composition according to claim 1, 5 or 6 comprising a concentrate according to any one of claims 2 to 4 in a concentration of 0.5 to 100 g/L, preferably 1 to 50 g/L, more preferably 2 to 30 g/L, most preferably 2 to 20 g/L.

8. Method for cleaning and/or disinfecting objects, preferably textiles made of synthetic and/or natural materials, wherein the objects are brought into contact with a disinfective cleaning composition or diluted concentrate according to any one of claims 1 to 7 in an effective concentration, at a temperature and for a time suitable for cleaning and/or disinfecting said objects.

9. Method according to claim 8, wherein the contact temperature is 0 to 80, preferably 15 to 50, more preferably 20 to 45°C.

10. Method according to claim 8 or 9, wherein the objects are brought into contact with the concentrate in a concentration of 0.5 to 100 g/L, preferably 1 to 50 g/L, more preferably 2 to 30 g/L, most preferably 2 to 20 g/L aqueous solvent, preferably water.

11. Method according to any of claims 8 to 10, wherein the time of contact is at least 1 second, preferably at least 1, 5 or 20 seconds to at most 2, 3, 5, 10, 30 or 60 minutes, more preferably at least 1 second to 10 minutes or 5 seconds to 5 minutes, most preferably 5 seconds to 3 minutes or 20 seconds to 2 minutes.

12. Method according to any of claims 8 to 11, wherein component A of the concentrate is used in a concentration of 0.2 to 20, preferably 0.5 to 10, more preferably 0.5 to 5 g/L and component B of the concentrate is used in a concentration of 1 to 30, preferably 2 to 15 g/L.

13. Method according to any of claims 8 to 12 for killing virus, preferably for killing virus H1N1, on objects, preferably textiles made of synthetic and/or natural materials, preferably wherein
(i) the contact temperature is 0 to 50, more preferably 5 to 30, most preferably 10 to 25°C, and/or
(ii) the time of contact is at least 1 to 10 minutes, preferably 5 seconds to 3 minutes, most preferably 20 seconds to 2 minutes.

14. Use of a disinfective cleaning composition and/or concentrate according to any one of claims 1 to 7 and/or a method of any of claims 8 to 12 for cleaning and/or disinfecting objects, preferably textiles made of synthetic and/or natural materials, more preferably selected from clothes, linen, towels, shoe ware, table cloths, drapes and bags.

15. Use of a disinfective cleaning composition and/or concentrate according to any one of claims 1 to 7 and/or a method of any of claims 8 to 13 for killing virus, preferably the virus H1N1 on objects, preferably textiles made of synthetic and/or natural materials, more preferably selected from clothes, linen, towels, shoe ware, table cloths, drapes and bags.
